Europäisches Patentamt

European Patent Office

Office européen des brevets

(19)

(11) Numéro de publication : **0 433 129 B1**

(12) **FASCICULE DE BREVET EUROPEEN**

(45) Date de publication du fascicule du brevet :
27.10.93 Bulletin 93/43

(21) Numéro de dépôt : **90403438.6**

(22) Date de dépôt : **04.12.90**

(51) Int. Cl.⁵ : **C07C 19/08,** C07C 17/38

(54) **Purification du 1,1-dichloro-1-fluoroethane.**

(30) Priorité : **15.12.89 US 451074**

(43) Date de publication de la demande :
**19.06.91 Bulletin 91/25**

(45) Mention de la délivrance du brevet :
**27.10.93 Bulletin 93/43**

(84) Etats contractants désignés :
**DE ES FR GB IT NL**

(56) Documents cités :
**EP-A- 0 389 334**
**US-A- 2 894 044**
**US-A- 3 026 359**

(73) Titulaire : **ELF ATOCHEM S.A.**
**4 & 8, Cours Michelet La Défense 10**
**F-92800 Puteaux (FR)**

(72) Inventeur : **Tung, Hsueh Sung**
**75 Brookland Drive**
**Williamsville, New York 14221 (US)**
Inventeur : **Smith, Addison Miles**
**80 Berryman Drive**
**Amherst, New York 14226 (US)**

(74) Mandataire : **Leboulenger, Jean et al**
**ATOCHEM Département Propriété Industrielle**
**Cédex 42- La Défense 10**
**F-92091 Paris la Défense (FR)**

## Description

La présente invention concerne la purification du 1,1-dichloro-1-fluoroéthane pour en éliminer les composés insaturés (notamment le dichloroacétylène et le chlorure de vinylidène).

## ARRIERE-PLAN DE L'INVENTION

Le trichlorofluorométhane (connu dans la technique sous le nom de CFC-11) est un produit commercial utilisé comme agent gonflant pour les mousses de polyuréthanne rigides. Le 1,1,2-trichloro-1,2,2-trifluoroéthane (connu dans la technique sous le nom de CFC-113) est aussi un produit commercial utilisé comme solvant pour nettoyer les cartes à circuit intégré. Actuellement, on considère que le 1,1-dichloro-1-fluoroéthane (connu dans la technique sous le nom de HCFC-141b) est un remplaçant du CFC-11 et du CFC-113, car le HCFC-141b ne décompose pas l'ozone de la stratosphère dans la même mesure que le CFC-11 ou le CFC-113. Comme la demande en HCFC-141b va augmenter considérablement dans l'avenir, on a besoin de procédés commercialement viables pour la préparation du HCFC-141b pur.

On peut produire le HCFC-141b en faisant réagir le chlorure de vinylidène avec le fluorure d'hydrogène anhydre. La réaction est exprimée par l'équation suivante :

$$CH_2=CCl_2 + HF \rightarrow CH_3CFCl_2$$

On peut aussi produire le HCFC-141b en faisant réagir le 1,1,1-trichloroéthane avec le fluorure d'hydrogène anhydre. La réaction est exprimée par l'équation suivante :

$$CH_3CCl_3 + HF \rightarrow CH_3CFCl_2 + HCl$$

Lorsqu'on utilise du chlorure de vinylidène, du dichloroacétylène est présent comme impureté dans la matière première et survit à la réaction de préparation du 1,1-dichloro-1-fluoroéthane. Le dichloroacétylène traverse aussi les processus de purification normaux comme la distillation et se retrouve dans le produit final.

Que l'on prépare le 1,1-dichloro-1-fluoroéthane en utilisant du chlorure de vinylidène ou du 1,1,1-trichloroéthane avec le fluorure d'hydrogène anhydre, du chlorure de vinylidène est toujours présent sous forme d'impureté dans le produit final. Lorsqu'on utilise du chlorure de vinylidène comme substance de départ, des quantités résiduelles de chlorure de vinylidène n'ayant pas réagi sont présentes dans le produit final. Lorsqu'on utilise le 1,1,1-trichloroéthane comme matière première, la décomposition thermique du 1,1,1-trichloroéthane conduit à la formation de petites quantités de chlorure de vinylidène qui sont présentes dans le produit final. Comme le chlorure de vinylidène a un point d'ébullition de 31,5°C et que le 1,1-dichloro-1-fluoroéthane a un point d'ébullition de 32°C, il est difficile d'éliminer le chlorure de vinylidène du 1,1-dichloro-1-fluoroéthane.

Le dichloroacétylène est un composé très instable et extrêmement toxique. Le dichloroacétylène est mutagène et a un fort potentiel cancérogène. La concentration maximale admissible (TLV) de l'exposition au DCA est de 0,1 partie par million. Le dichloroacétylène présent dans le 1,1-dichloro-1-fluoroéthane après la distillation varie de 5 à 20 parties par million.

Le chlorure de vinylidène est aussi un composé très toxique. Une exposition excessive peut provoquer des lésions aux reins, au foie ou au système nerveux central. La TLV pour l'exposition au chlorure de vinylidène est de 5 parties par million. Le chlorure de vinylidène présent dans le 1,1-dichloro-1-fluoroéthane après la distillation varie de 500 à 1 200 parties par million. Bien que les études de toxicité à grande échelle pour le 1,1-dichloro-1-fluoroéthane soient actuellement incomplètes, le "Panel for Advancement of Fluorocarbon Test" (PAFT II), qui définit la pureté du produit 1,1-dichloro-1-fluoroéthane, a établi les spécifications actuelles du dichloroacétylène et du chlorure de vinylidène comme étant respectivement de 1 partie par million et de 200 parties par million.

Ainsi, un besoin existe pour un procédé permettant d'éliminer ou de réduire les quantités de dichloroacétylène et de chlorure de vinylidène dans le produit 1,1-dichloro-1-fluoroéthane.

La purification des hydrocarbures halogénés a fait l'objet de nombreuses références. En général, ces procédés concernent l'élimination de sous-produits de réaction qui sont difficiles à éliminer par les procédés ordinaires.

Le brevet U.S. 2 879 228 décrit que l'on élimine les impuretés hydrogénées par mise en contact avec de l'alumine ou de la silice à une température de 100 à 250°C. Les impuretés sont absorbées par l'alumine ou la silice pour laisser un hydrocarbure perfluoré purifié.

La demande de brevet japonais 48-103 502 décrit l'utilisation de solutions de carbonate de sodium pour le traitement des perchlorofluoroalcanes comme le trichlorotrifluoroéthane à 25-60°C en vue d'éliminer les hydrocarbures halogénés hydrogénés.

Le brevet U.S. 3 696 156 décrit l'emploi d'alumine avec 0,1 à 5% d'un métal alcalin ou d'un métal alcalino-terreux pour éliminer les impuretés insaturées jusqu'à une valeur inférieure à 2 parties par million.

Le brevet DE 3 017 531 décrit la purification de réfrigérants contaminés comme le trichlorotrifluoroéthane par contact avec de l'alumine et un métal alcalino-terreux.

Le brevet DE 3 311 751 décrit qu'une zéolithe ayant une grosseur de pore de 0,4 à 1 nm est utile pour éliminer les halogènes et les halogénures minéraux des hydrocarbures fluorochlorés comme le trichlorotrifluoroéthane.

Le brevet japonais 83-035 737 décrit la régénération d'une zéolithe utilisée pour purifier et sécher le trichloroéthane.

Le brevet russe SU 743 985 décrit la purification des solvants organiques chlorés et fluorés en les faisant passer en phase vapeur sur du charbon actif en présence de pentoxyde de phosphore.

Le brevet US 4 849 558 décrit que l'on peut purifier les solvants chlorofluorocarbonés comme le 1,1,2-trichloro-1,2,2-trifluoroéthane en éliminant le dioxyde de soufre au contact d'alumine ou de zéolithes.

Confronté au présent problème, on a considéré différents procédés de purification, y compris l'adsorption sélective. L'adsorption sélective nécessite un équilibre adéquat entre la polarité et la grosseur de pore de l'adsorbant et les moments dipolaires et les tailles moléculaires des adsorbats et du solvant. La polarité et la grosseur de pore de l'adsorbant doivent correspondre aux moments dipolaires et aux tailles moléculaires du dichloroacétylène et du chlorure de vinylidène et ne pas correspondre au moment dipolaire et à la taille moléculaire du 1,1-dichloro-1-fluoroéthane afin d'adsorber le dichloroacétylène et le chlorure de vinylidène sélectivement par rapport au 1,1-dichloro-1-fluoroéthane. Le moment dipolaire du dichloroacétylène est estimé à 0,07 debye et la taille moléculaire est de 60 angströms$^3$. Le moment dipolaire du chlorure de vinylidène est de 1,34 debye et la taille moléculaire est calculée comme étant de 70 angströms$^3$. Le moment dipolaire du 1,1-dichloro-1-fluoroéthane est de 2,14 debye et la taille moléculaire est de 82 angströms$^3$.

On pouvait penser que les tamis moléculaires qui sont faits d'un mélange d'oxyde d'aluminium et de dioxyde de silicium et qui ont une grosseur de pore nominale allant de 3 à 5 angströms pourraient correspondre aux moments dipolaires et aux tailles moléculaires du dichloroacétylène et du chlorure de vinylidène et ne pas correspondre au 1,1-dichloro-1-fluoroéthane, de manière à adsorber sélectivement le dichloroacétylène et le chlorure de vinylidène par rapport au 1,1-dichloro-1-fluoroéthane. Comme on l'indique ci-dessous dans les exemples comparatifs, les tamis moléculaires sont incapables d'adsorber sélectivement le dichloroacétylène et le chlorure de vinylidène.

On pouvait penser aussi que la silicalite qui est faite principalement de silice et a une grosseur de pore d'environ 5 à 6 angströms correspondrait aux moments dipolaires et aux tailles moléculaire du dichloroacétylène et du chlorure de vinylidène et ne correspondrait pas au 1,1-dichloro-1-fluoroéthane, de manière à adsorber sélectivement le dichloroacétylène et le chlorure de vinylidène par rapport au 1,1-dichloro-1-fluoroéthane. Comme l'indiquent ci-dessous les exemples comparatifs, la silicalite est incapable d'adsorber sélectivement le dichloroacétylène et le chlorure de vinylidène.

## RESUME DE L'INVENTION

On a maintenant découvert de façon surprenante que le charbon actif est capable d'adsorber sélectivement le dichloroacétylène et le chlorure de vinylidène par rapport au 1,1-dichloro-1-fluoroéthane.

La présente invention a donc pour objet un procédé de purification du 1,1-dichloro-1-fluoroéthane obtenu par réaction du fluorure d'hydrogène anhydre avec du 1,1,1-trichloroéthane ou du chlorure de vinylidène contenant du dichloroacétylène, caractérisé en ce que l'on fait passer le 1,1-dichloro-1-fluoroéthane sur du charbon actif pour éliminer sensiblement les impuretés insaturées.

Le procédé selon l'invention permet notamment de réduire les teneurs en dichloroacétylène et en chlorure de vinylidène dans le 1,1-dichloro-1-fluoroéthane pour satisfaire aux spécifications actuelles définies par le "Panel for Advancement of Fluorocarbon Test".

D'autres buts et avantages de l'invention apparaîtront à la lecture de la description qui suit.

## DESCRIPTION DETAILLEE DE L'INVENTION

Le 1,1-dichloro-1-fluoro-éthane dont la purification fait l'objet de la présente invention provient de la réaction de fluorure d'hydrogène anhydre avec du 1,1,1-trichloroéthane ou du chlorure de vinylidène. Lorsqu'on utilise le 1,1,1-trichloroéthane comme substance de départ, on peut par exemple faire réagir 17 parties en poids de fluorure d'hydrogène et 3,73 parties en poids de 1,1,1-trichloroéthane à 110°C pendant 15 minutes pour former le 1,1-dichloro-1-fluoroéthane; voir le brevet U.S. 3 833 676, qui est cité ici à titre de référence dans la mesure nécessaire pour compléter cette description. Lorsqu'on utilise le chlorure de vinylidène comme substance de départ, on peut par exemple faire réagir quatre moles de fluorure d'hydrogène avec le chlorure de vinylidène pendant trois heures à 65°C pour former le 1,1-dichloro-1-fluoroéthane; voir A.L. Henne et coll., J. Am. Chem. Soc. 65, 1272 (1943). Le fluorure d'hydrogène anhydre, le 1,1,1-trichloroéthane et le chlorure de vinylidène disponibles dans le commerce peuvent être utilisés pour ces préparations. Comme on l'a indiqué plus haut, du dichloroacétylène est présent comme impureté dans le chlorure de vinylidène et survit au processus de préparation du 1,1-dichloro-1-fluoroéthane.

De préférence, après la préparation du 1,1-dichloro-1-fluoroéthane, on distille le 1,1-dichloro-1-fluoroéthane résultant pour éliminer les impuretés comme le fluorure d'hydrogène et les produits de point d'ébullition élevés, notamment les goudrons.

L'étape de purification du présent procédé fait intervenir le passage du 1,1-dichloro-1-fluoroéthane sur du charbon actif pour éliminer essentiellement les impuretés insaturées. De préférence, les impuretés insaturées éliminées sont le chlorure de vinylidène et le dichloroacétylène. Le charbon actif disponible dans le commerce est utile dans le présent procédé. Le rendement de l'adsorption et la capacité d'adsorption du charbon actif dépendent de la granulométrie du charbon actif dans un système à flux dynamique. De préférence, le charbon actif a une granulométrie d'environ 0,005 millimètres à environ 10 millimètres. On préfère encore que le charbon actif ait une granulométrie d'environ 0,04 millimètre à environ 5 millimètres. On préfère tout particulièrement que le charbon actif ait une granulométrie d'environ 0,1 millimètre à environ 2 millimètres. La capacité d'adsorption d'un charbon actif donné peut également être améliorée par élimination de la teneur en cendres du charbon ; cela peut être fait par une technique standard comme, par exemple, un lavage acide.

Un charbon actif ayant une granulométrie de 0,595 millimètre x 1,68 millimètres (12 x 30 mesh) est disponible auprès de Calgon Corporation sous le nom de charbon Calgon PCB (à base de noix de coco de Pittsburgh). Un autre charbon actif ayant une granulométrie de 0,105 millimètre x 0,595 millimètre (30 x 140 mesh) est disponible auprès de Calgon Corporation sous le nom de charbon Calgon PCB (à base de noix de coco de Pittsburgh). Un autre charbon actif ayant une granulométrie de 0,42 millimètre x 1,68 millimètres (12 x 40 mesh) est disponible auprès de Calgon Corporation sous le nom de charbon Calgon CAL (à base de charbon bitumineux).

Que l'on utilise du 1,1,1-trichloroéthane ou du chlorure de vinylidène dans la préparation du 1,1-dichloro-1-fluoroéthane, après la distillation, le 1,1-dichloro-1-fluoroéthane contient d'environ 500 à environ 1 200 parties par million de chlorure de vinylidène. Le 1,1-dichloro-1-fluoroéthane préparé à partir de chlorure de vinylidène contient d'environ 5 à environ 20 parties par million de dichloroacétylène. Grâce à la mise en oeuvre du présent procédé, les quantités de dichloroacétylène dans le 1,1-dichloro-1-fluoroéthane sont réduites considérablement, voire totalement éliminées, et les quantités de chlorure de vinylidène dans le 1,1-dichloro-1-fluoroéthane sont réduites à une concentration inférieure à environ 200 parties par million.

Après la mise en oeuvre du présent procédé, on peut ensuite distiller le 1,1-dichloro-1-fluoroéthane résultant.

La purification s'effectue typiquement à la température ambiante et sous la pression atmosphérique, ce qui fait que le 1,1-dichloro-1-fluoroéthane est en phase liquide. Contrairement aux procédés de purification connus, où le charbon actif est préchauffé à une température de 300°C à 400°C, le chauffage n'est pas nécessaire dans le présent procédé de purification. Comme le charbon actif se réchauffe pendant l'étape de purification, on refroidit généralement le charbon actif afin de le maintenir à la température ambiante. De plus, à l'inverse des procédés connus dans lesquels la purification s'effectue en présence de pentoxyde de phosphore, la présence de pentoxyde de phosphore n'est pas nécessaire dans la présente méthode de purification.

La purification peut être menée d'une manière discontinue ou continue.

Le 1,1-dichloro-1-fluoroéthane purifié produit par le présent procédé est utile comme agent gonflant pour la production de mousses de polyuréthanne rigides. Voir, par exemple, les brevets U.S. 4 271 273, 4 652 589, 4 686 240, 4 699 932, 4 701 474, 4 717 518 et 4 727 094. Le 1,1-dichloro-1-fluoroéthane purifié est aussi utile pour nettoyer les surfaces solides par traitement des surfaces d'une manière connue dans la technique, comme par trempage ou par pulvérisation.

La présente invention est illustrée plus en détail par les exemples non limitatifs suivants.

EXEMPLES COMPARATIFS A-E ET EXEMPLE 1

Pour l'exemple comparatif A, on utilise un tamis moléculaire fourni par Union Carbide, fait d'un mélange d'oxyde d'aluminium et de dioxyde de silicium et ayant une grosseur de pore nominale de 3 angströms. Pour l'exemple comparatif B, on utilise un tamis moléculaire fourni par Union Carbide, fait d'un mélange d'oxyde d'aluminium et de dioxyde de silicium et ayant une grosseur de pore nominale de 4 angströms. Pour l'exemple comparatif C, on utilise un tamis moléculaire fourni par Union Carbide, fait d'un mélange d'oxyde d'aluminium et de dioxyde de silicium et ayant une grosseur de pore nominale de 5 angströms. Pour l'exemple comparatif D, on utilise un type de silicalite SP-115 fourni par Union Carbide et fait de silice liée à de l'aluminium. Pour l'exemple comparatif E, on utilise un type de silicalite SR-115 fourni par Union Carbide et fait de silice liée à de la silice.

Pour l'exemple 1, on utilise du charbon actif fourni par Calgon Corporation, désigné par PCB (à base de noix de coco de Pittsburgh) et ayant une granulométrie de 0,595 millimètre x 1,68 millimètres (12 x 30 mesh).

L'adsorbant est séché dans une étuve à vide à une température comprise entre 110 et 150°C pendant 24 à 72 heures. Après séchage, l'adsorbant est utilisé pour garnir une colonne de verre de longueur 48 centimètres et de diamètre 3,9 centimètres. Le sommet de la colonne d'adsorption est équipé d'une ampoule à brome de 250 millilitres et d'un réfrigérant de 46 centimètres de longueur. On utilise un 1,1-dichloro-1-fluoroéthane impur préparé à partir de chlorure de vinylidène et de fluorure d'hydrogène. Le 1,1-dichloro-1-fluoroéthane contient 875 parties par million de chlorure de vinylidène et 13 parties par million de dichloroacétylène. On fait passer dans la colonne d'adsorption environ 250 à 500 millilitres du 1,1-dichloro-1-fluoroéthane impur.

Pour les exemples comparatifs, on recueille les 50 à 100 premiers millilitres de 1,1-dichloro-1-fluoroéthane élués dans la colonne d'adsorption et on dose la concentration de chlorure de vinylidène et/ de dichloroacétylène; les résultats sont indiqués sur le tableau I ci-dessous. On recueille aussi les 50 derniers millilitres et on les analyse; comme prévu, les quantités de chlorure de vinylidène sont plus élevées dans les 50 derniers millilitres que dans les 50 premiers millilitres. Pour l'exemple 1, on recueille et on analyse les 50 à 100 premiers et les 50 à 100 derniers millilitres des échantillons élués; les résultats sont indiqués sur le tableau I ci-dessous.

L'abréviation utilisée pour le chlorure de vinylidène est CV2 et on l'exprime en parties par million (ppm) et l'abréviation utilisée pour le dichloroacétylène est DCA et on l'exprime en parties par million (ppm) sur le tableau I.

Dans le 1,1-dichloro-1-fluoroéthane recueilli, on dose d'abord le chlorure de vinylidène et, si la proportion est inférieure à 200 parties par million, on dose alors le dichloroacétylène dans l'échantillon. Si la proportion est supérieure à 200 parties par million, on ne dose pas le dichloroacétylène dans l'échantillon (sur le tableau, on utilise l'abréviation NA). ND signifie non décelable.

## TABLEAU I

| EX./COMP. | ADSORBANT | CV2(ppm) | DCA(ppm) |
|---|---|---|---|
| HCFC-141b | -- | 875 | 13 |
| A | tamis moléculaire 3A | 651 | NA |
| B | tamis moléculaire 4A | 1021 | NA |
| C | tamis moléculaire 5A | 585 | NA |
| D | silicalite SP-115 | 410 | NA |
| E | silicalite SR-115 | 380 | NA |
| 1 | charbon actif | ND | ND |

Le tamis moléculaire de l'exemple comparatif B adsorbe plus le 1,1-dichloro-1-fluoroéthane que le chlorure de vinylidène, ce qui conduit à une concentration plus grande en chlorure de vinylidène dans l'échantillon élué que dans l'échantillon initial. Pour l'exemple 1, on ne décèle pas de chlorure de vinylidène ou de dichloroacétylène, que ce soit dans les 50 premiers ou dans les 50 derniers millilitres d'échantillon élué.

## EXEMPLE 2

On reprend l'exemple 1, sauf que l'on remplace l'ampoule à brome par un autre réfrigérant de 46 centimètres de longueur. On utilise une petite pompe pour pomper en continu le 1,1-dichloro-1-fluoroéthane impur dans l'appareil. On utilise 229 grammes du charbon actif utilisé dans l'exemple 1 pour garnir la colonne de verre. On prépare un échantillon de 1,1-dichloro-1-fluoroéthane impur pour qu'il contienne 3080 parties par million de chlorure de vinylidène, ce qui est une concentration supérieure à la normale. Tout en pompant et en éluant en continu l'échantillon de 1,1-dichloro-1-fluoroéthane impur dans la garniture de lit de charbon, à environ 29 grammes/minute, on prélève des échantillons instantanés et on les analyse pour déterminer la quantité de chlorure de vinylidène. On ne fait aucun dosage du dichloroacétylène dans cette expérience. Les résultats sont indiqués sur le tableau II ci-dessous.

## TABLEAU II

| POIDS D'ELUTION DU HCFC-141b IMPUR (grammes) | CV2 (ppm) |
|---|---|
| 250 | 300 |
| 480 | 600 |
| 720 | 1 100 |
| 960 | 1 500 |

### EXEMPLE 3

On reprend l'exemple 2, sauf que l'on utilise un charbon actif fourni par Calgon Corporation, désigné par PCB (à base de noix de coco de Pittsburgh) et ayant une granulométrie de 0,105 millimètre x 0,595 millimètre (30 x 140 mesh). On garnit la colonne avec 222 grammes de charbon actif. On pompe en continu à travers le lit de charbon un 1,1-dichloro-1-fluoroéthane impur contenant 875 parties par million de chlorure de vinylidène et 13 parties par million de dichloroacétylène, qui constituent des quantités normales, à environ 18,2 grammes/minute. On recueille pour analyse des échantillons instantanés élués dans la colonne. Les résultats sont indiqués sur le tableau III ci-dessous.

## TABLEAU III

| POIDS D'ELUTION DU HCFC-141b IMPUR (grammes) | CV2(ppm) | DCA(ppm) |
|---|---|---|
| HCFC-141b initial | 875 | 13 |
| 23 | 40 | ND |
| 193 | 34 | ND |
| 427 | 29 | ND |
| 624 | 28 | ND |
| 801 | 12 | ND |

On ne décèle de dichloroacétylène dans aucun des échantillons purifiés. De plus, la concentration du chlorure de vinylidène dans chaque échantillon est bien inférieure à 200 parties par million.

### EXEMPLE 4

On reprend l'exemple 3, sauf que l'on utilise un charbon actif à base de charbon bitumineux fourni par Calgon Corporation, sous la désignation CAL et ayant une granulométrie de 0,42 millimètre x 1,68 millimètres (12 x 40 mesh). On garnit la colonne de 230 grammes de ce charbon actif. On pompe en continu à travers le garnissage de lit de charbon le même 1,1-dichloro-1-fluoroéthane impur que celui utilisé dans l'exemple 3. On recueille les échantillons instantanés élués dans la colonne et on les analyse pour doser le dichloroacétylène et le chlorure de vinylidène. Les résultats figurent au tableau IV ci-dessous.

EP 0 433 129 B1

## TABLEAU IV

| POIDS D'ELUTION DU HCFC-141b IMPUR (grammes) | CV2(ppm) | DCA(ppm) |
|---|---|---|
| HCFC-141b initial | 875 | 13 |
| 105 | 7 | ND |
| 342 | 47 | ND |
| 728 | 120 | ND |
| 994 | 154 | ND |

Bien que ce charbon actif soit efficace pour éliminer le dichloroacétylène, il n'est pas aussi efficace pour éliminer le chlorure de vinylidène.

Ayant décrit l'invention en détail à l'aide de ses modes de réalisation préférés, il semblera évident que des modifications et des variations sont possibles sans que l'on sorte du cadre de l'invention tel qu'il est défini dans les revendications annexées.

**Revendications**

1. Procédé de purification du 1,1-dichloro-1-fluoroéthane obtenu par réaction du fluorure d'hydrogène anhydre avec du 1,1,1-trichloroéthane ou du chlorure de vinylidène contenant du dichloroacétylène, caractérisé en ce que l'on fait passer ledit 1,1-dichloro-1-fluoroéthane sur du charbon actif pour éliminer sensiblement les impuretés insaturées.

2. Procédé selon la revendication 1, dans lequel, avant son passage sur le charbon actif, on distille ledit 1,1-dichloro-1-fluoroéthane.

3. Procédé selon la revendication 1 ou 2, dans lequel, après passage sur le charbon actif, on soumet le ledit 1,1-dichloro-1-fluoroéthane à une distillation.

4. Procédé selon l'une des revendications 1 à 3, dans lequel lesdites impuretés insaturées sont le dichloroacétylène et le chlorure de vinylidène.

5. Procédé selon l'une des revendications 1 à 4, dans lequel ledit charbon actif a une granulométrie d'environ 0,005 millimètre à environ 10 millimètres.

6. Procédé selon la revendication 5, dans lequel ledit charbon actif a une granulométrie d'environ 0,04 millimètre à environ 5 millimètres et de préférence d'environ 0,1 millimètre à environ 2 millimètres.

7. Procédé selon la revendication 2, dans lequel, après passage sur charbon actif, le dichloroacétylène est entièrement éliminé du 1,1-dichloro-1-fluoroéthane.

8. Procédé selon la revendication 2, dans lequel, après passage sur charbon actif, la concentration en chlorure de vinylidène dans le 1,1-dichloro-1-fluoroéthane est inférieure à environ 200 parties par million.

9. Procédé selon l'une des revendications 1 à 8, dans lequel ledit charbon actif a une granulométrie d'environ 0,595 millimètre à environ 1,68 millimètres.

10. Procédé selon l'une des revendications 1 à 8, dans lequel ledit charbon actif a une granulométrie d'environ 0,105 millimètre à environ 0,595 millimètre.

11. Procédé selon l'une des revendications 1 à 8, dans lequel ledit charbon actif a une granulométrie d'environ 0,42 millimètre à environ 1,68 millimètres.

7

## Claims

1. Process for the purification of 1,1-dichloro-1-fluoroethane obtained by reacting anhydrous hydrogen fluoride with 1,1,1-trichloroethane or vinylidene chloride containing dichloroacetylene, characterised in that the said 1,1-dichloro-1-fluoroethane is passed over active charcoal in order substantially to remove the unsaturated impurities.

2. Process according to Claim 1, in which, before being passed over active charcoal, the said 1,1-dichloro-1-fluoroethane is distilled.

3. Process according to Claim 1 or 2, in which, after the passage over active charcoal, the said 1,1-dichloro-1-fluoroethane is subjected to a distillation.

4. Process according to one of Claims 1 to 3, in which the said unsaturated impurities are dichloroacetylene and vinylidene chloride.

5. Process according to one of Claims 1 to 4, in which the said active charcoal has a particle size of approximately 0.005 millimetre to approximately 10 millimetres.

6. Process according to Claim 5, in which the said active charcoal has a particle size of approximately 0.04 millimetre to approximately 5 millimetres, and preferably approximately 0.1 millimetre to approximately 2 millimetres.

7. Process according to Claim 2, in which, after the passage over active charcoal, the dichloroacetylene is completely removed from the 1,1-dichloro-1-fluoroethane.

8. Process according to Claim 2, in which, after the passage over active charcoal, the concentration of vinylidene chloride in the 1,1-dichloro-1-fluoroethane is less than approximately 200 parts per million.

9. Process according to one of Claims 1 to 8, in which the said active charcoal has a particle size of approximately 0.595 millimetre to approximately 1.68 millimetres.

10. Process according to one of Claims 1 to 8, in which the said active charcoal has a particle size of approximately 0.105 millimetre to approximately 0.595 millimetre.

11. Process according to one of Claims 1 to 8, in which the said active charcoal has a particle size of approximately 0.42 millimetre to approximately 1.68 millimetres.


## Patentansprüche

1. Verfahren zur Reinigung von 1,1-Dichlor-1-fluorethan, das durch die Reaktion von wasserfreiem Fluorwasserstoff mit 1,1,1-Trichlorethan oder mit dichloracetylenhaltigem Vinylidenchlorid gewonnen wurde, dadurch gekennzeichnet, daß besagtes 1,1-Dichlor-1-fluorethan über Aktivkohle geleitet wird, um die ungesättigten Verunreinigungen in erheblichem Maße zu beseitigen.

2. Verfahren nach Anspruch 1, in dem besagtes 1,1-Dichlor-1-fluorethan vor dem Überleiten über die Aktivkohle destilliert wird.

3. Verfahren nach Anspruch 1 oder 2, in dem besagtes 1,1-Dichlor-1-fluorethan nach dem Überleiten über die Aktivkohle destilliert wird.

4. Verfahren nach einer der Ansprüche 1 bis 3, in dem die besagten ungesättigten Verunreinigungen das Dichloracetylen und das Vinylidenchlorid sind.

5. Verfahren nach einer der Ansprüche 1 bis 4, in dem die besagte Aktivkohle eine Korngröße von ungefähr 0,005 Millimeter bis ungefähr 10 Millimeter aufweist.

6. Verfahren nach Anspruch 5, in dem die besagte Aktivkohle eine Korngröße von ungefähr 0,04 Millimeter bis ungefähr 5 Millimeter und vorzugsweise von ungefähr 0,1 Millimeter bis ungefähr 2 Millimeter aufweist.

7. Verfahren nach Anspruch 2, in dem das Dichloracetylen nach dem Überleiten über die Aktivkohle vollständig aus dem 1,1-Dichlor-1-fluorethan entfernt ist.

8. Verfahren nach Anspruch 2, in dem die Konzentration von Vinylidenchlorid im 1,1-Dichlor-1-fluorethan nach dem Überleiten über die Aktivkohle weniger als ungefähr 200 ppm beträgt.

9. Verfahren nach einer der Ansprüche 1 bis 8, in dem die besagte Aktivkohle eine Korngrösse von ungefähr 0,595 Millimeter bis ungefähr 1,68 Millimeter aufweist.

10. Verfahren nach einer der Ansprüche 1 bis 8, in dem die besagte Aktivkohle eine Korngrösse von ungefähr 0,105 Millimeter bis ungefähr 0,595 Millimeter aufweist.

11. Verfahren nach einer der Ansprüche 1 bis 8, in dem die besagte Aktivkohle eine Korngrösse von ungefähr 0,42 Millimeter bis ungefähr 1,68 Millimeter aufweist.